# EUROPEAN PATENT APPLICATION

(11) **EP 3 081 653 A1**
(43) Date of publication of application: **19.10.2016**
(21) Application number: 15752094.1
(22) Date of filing: 02.02.2015
(51) Int. Cl.: C12Q 1/68

(54) **METHOD FOR DETECTING NUCLEIC ACID USING ASYMMETRIC ISOTHERMAL AMPLIFICATION OF NUCLEIC ACID AND SIGNAL PROBE**

(30) Priority: 21.02.2014 KR 20140020322
(71) Applicant: Dxgene Inc., Seoul 138-851 (KR)
(72) Inventor: KIM, MinHwan, Seoul 133-866 (KR)
(74) Representative: Loo, Chi Ching
(86) International application number: PCT/KR2015/001070
(87) International publication number: WO 2015/126078

(57) **Abstract**

The present invention relates to a method for detecting target nucleic acids by asymmetric isothermal amplification of the target nucleic acids and a signal probe using an external primer set and different concentration ratio of forward and reverse of DNA-RNA-DNA hybrid inner primers and a DNA-RNA-DNA hybrid signal probe. The method according to the present invention can be used to efficiently detect the signal of the probe compared to the conventional symmetric isothermal target and probe amplification method that using the same concentration ratio of primers, so that it can be applied to various genome projects, detection and identification of a pathogen, detection of gene modification producing a predetermined phenotype, detection of hereditary diseases or determination of sensibility to diseases, and estimation of gene expression. Thus, it is useful for molecular biological studies and disease diagnosis.

## Description

### FIELD OF INVENTION

The present invention relates to a method for isothermal amplification of nucleic acids and a signal probe, and a method for detecting target nucleic acids by isothermal amplification of signal probe. More particularly, the present invention relates to a method for detecting target nucleic acids rapidly by simultaneously amplifying target nucleic acids and a signal probe using an external primer set, a DNA-RNA-DNA hybrid primer set and a DNA-RNA-DNA hybrid signal probe. More particularly, the present invention relates to a method for detecting target nucleic acids by asymmetrically amplifying target nucleic acids using different concentration ratio of forward and reverse of DNA-RNA-DNA hybrid inner primers simultaneously amplifying signal by a DNA-RNA-DNA hybrid signal probe which has a complementary nucleotide sequence to excessively amplified DNA that are asymmetrically amplifying by using the excess amount of the DNA-RNA-DNA hybrid primer.

### BACKGROUND OF INVENTION

A nucleic acids amplification technique is very useful for detecting and analyzing a small quantity of nucleic acid. A high sensitivity to target nucleic acids in the nucleic acids amplification enables to develop a technology of detecting specific nucleic acids in a field of gene detection for diagnosis and analysis of infectious disease and genetic disease and in forensic field. Based on such method for detecting nucleic acid, the various methods which can execute a very sensitive diagnosis and analysis have been developed (Belkum, Current Opinion in Pharmacology, 3:497, 2003). Detection of nucleic acid is achieved by complementarity of DNA strands and the ability of single stranded nucleic acid to form double stranded hybrid molecules in vitro. Due to this ability, it is possible to detect specific nucleic acids in a sample (Barry et al, Current Opinion in Biotechnology, 12:21, 2001).

A probe used in detection of nucleic acid is composed of specific sequences capable of hybridize with a target sequence of a nucleic acid present in a sample. The probe is read by chemical materials, immune chemicals, fluorescent materials or radioisotopes. Generally, probes are composed to include fluorescent materials capable of reading DNA hybridization and short fragment of nucleic acids having complementary sequence to target nucleic acids, or markers or report molecules such as biotin and digoxygenin.

However, the above mentioned methods have problems in that they cannot detect a short sequence on the chromosomal DNA, result in low copy numbers and has a difficulty to solve the problem of the limited copy number of modified allele of wild-type gene. Another problem of the method is related to *in vitro* or *in situ* environmental conditions, which limit physical interaction among a target sequence, a chemical, a probe and an another molecular structures.

The method for detection of target nucleic acid is classified into three categories, that is, (1) target sequence amplification in which target nucleic acids are amplified, (2) probe amplification in which probe molecules are amplified, and (3) signal amplification in which a probe signal level is increased by probe hybridization technique or multiplex ligation-dependent probe amplification technique.

In vitro nucleic acid amplification techniques have been used as leading methods in detecting and analyzing a small quantity of nucleic acid. Among them, PCR (polymerase chain reaction) is most widely used as a nucleic acid amplification technique which uses repeated cycles of primer-dependent nucleic acid synthesis using each strand of a complementary sequence as a template and thus copies of each strand of a complementary sequence are synthesized. However, in order to carry out PCR, a pre-programmed thermal cycling instrument is needed. Also, PCR technique has the following shortcomings: it costs a lot; it has a relatively low specificity; performance procedure should be extremely standardized to reproduce RCR results.

In LCR (ligase chain reaction) which is another nucleic acid amplification technique, two adjacent oligonucleotides are hybridized with target nucleic acids, and ligated by a ligase, and then a probe formed through this method is amplified by temperature cycling together with a complementary nucleotide.

Since LCR has higher discriminatory power than primer extension using a primer, it shows higher allele specificity than that of PCR in genotyping point mutation. Among nucleic acid amplification techniques developed up until now, LCR has the highest specificity and it is the easiest method to perform because all of discrimination mechanisms are optimized. However, it has a shortcoming in that its reaction rate is the slowest and it requires many modified probes.

In methods using ligation such as LCR, genotyping can be performed by amplifying a primarily circularized padlock probe through DNA ligation accompanied by process of LCR or RCA (rolling circle amplification) technique without PCR target amplification (Qi et al, Nucleic Acids Res., 29:e116, 2001)

SDA (strand displacement amplification) is an amplification method of a target nucleic acid sequence and the complementary strand thereof in samples by strand displacement using endonuclease. This method uses a mixture containing nucleic acid polymerase, at least one primer complementary to 3'-terminal end of a target fragment and dNTPs (deoxynucleoside triphosphates) comprising of at least one substituted dNTP. Each primer has a sequence in 5 '-terminal end, which restriction endonuclease can recognize (Walker et al, Nucleic Acids Res., 29:1691, 1992).

As similar methods to SDA, there are SPIA (single primer isothermal amplification) technique using 5'-RNA-DNA-3' primer (US 6,251,639), ICAN(isothermal chimeric primer-initiated amplification of nucleic acid) technique using5'-DNA-RNA-3' primer (US 2005/0123950), Ribo primer technique using RNA primer (US 2004/0180361), technique using external primers and DNA-RNA-DNA hybrid primers (US 5,824,517) and etc., in which an extension of a primer using an RNA primer or an DNA-RNA-DNA hybrid primer followed by digesting of an RNA primer hybridized or an RNA of DNA-RNA-DNA hybrid primer with a template DNA by RNaseH, and then a new primer is extended by strand displacement.

TMA (transcription mediated amplification) is a target nucleic acid amplification technique using only one promoter-primer at a constant temperature, a constant ionic strength and a constant pH (Kwoh et al, Proc. Nat. Acad. Sci. USA, 86:1173, 1989). TMA comprises the step of combining a mixture composed of target nucleic acids and promoter-primer which is an oligonucleotide complementary to the 3'-terminal end of a target sequence for hybridization with the 3'-terminal of target nucleic acids or neighboring region thereof. The promoter-primer comprises a sequence of promoter region for RNA polymerase located in the 5'-terminal end of a complexing sequence. The promoter-primer and target sequence form a promoter- primer/ target sequence hybrid to extend DNA.

In the process of DNA extension of TMA technique, it is assumed that the 3'-terminal end of a target sequence is extended from the location close to a complex in which a promoter-primer is hybridized between a complexing sequence and a target sequence. A promoter-primer sequence produces a first DNA extension product to act as a template in an extension process forming a double stranded promoter sequence. The 3'-terminal end of the promoter-primer could be used as a template in the second DNA extension process. In the extension process, a double stranded nucleic acid complex is formed using a target sequence as a template. Subsequently, an RNA polymerase recognizing a promoter of the promoter-primer synthesizes RNA copies of target sequence.

NASBA (nucleic acid sequence-based amplification) technique comprises syntheses of single stranded RNA, single stranded DNA and double stranded DNA (Compton, Nature, 350:91, 1991). The single stranded RNA becomes the first template for the first primer, the single stranded DNA becomes the second template for the second primer, and the double stranded DNA becomes the third template in synthesis of copies for the first template.

The amplification method using heat cycle process such as PCR requires a heat block to reach "target" temperature of each cycle, and a delay time until the heat block reaches the target temperature, therefore it takes a long time until the amplification reaction is completed.

Since the method for isothermal amplification of target nucleic acids such as SDA, NASBA and TMA is performed at a constant temperature, it does not require a thermal cycling apparatus, and thus, it is easy to perform. However, the mentioned isothermal amplification methods of target nucleic acids have several disadvantages. The SDA method requires a specific region for a given restriction enzyme, so the application thereof is limited. The transcription-based amplification methods such NASBA and TMA require the binding between a polymerase promoter sequence and an amplification product by a primer, and this process tends to bring a non-specific amplification. Because of these disadvantages, the amplification mechanism of DNA target by transcription-based amplification methods has not been well-established.

Moreover, currently used amplification methods are disadvantageous in that there is a possibility of test samples being contaminated by the products of preceding amplification reaction, thereby causing non-specific target amplification. In order to prevent this, contamination detection methods of a sample solution which employ various means including physical means for decontaminating the sample in the last step of amplification reaction or before the beginning of target nucleic acid amplification, are being developed, but most of them make nucleic acid amplification procedure complicated.

As another method for detecting nucleic acid, there is a method for amplifying a signal, neither a target nucleic acid nor a probe. Among these methods, there is bDNA (branched DNA) amplification method using four sets of probes to capture a target nucleic acid (Ross et al, J. Virol. Method., 101 : 159, 2002). Hybrid capture method using signal amplification has sensitivity comparable to the method for directly detecting and amplifying a target nucleic acid, and uses an antibody or a luminous chemical for signal detection (van der Pol et al, J. Clinical Microbiol, 40:3564,2002; Nelson et al, Nucleic Acids Research, 24:4998, 1996).

Furthermore, there is CPT (cycling probe technology) as a method for amplifying a signal probe (Duck et al, BioTechniques, 9: 142, 1990). The method uses a DNA/RNA/DNA hybrid probe having a base sequence complementary to a target nucleic acid. In the method, a signal probe is amplified by repeating a procedure, in which when a signal probe is hybridized with a target nucleic acid, RNA region of the hybrid signal probe is digested by RNaseH and the digested hybrid signal probe is separated from the target nucleic acid, then an intact DNA-RNA-DNA hybrid probe is hybridized with the target nucleic acid. There is a FRET (fluorescent resonance energy transfer) based detection method that both ends of the DNA-RNA-DNA hybrid probe are labeled with a fluorescent dye and a quencher dye. (US patent application 2005/0214809) However, the above CPT method has disadvantages in that it has a relatively low amplification efficiency of 10² ∼10⁴, so it is difficult to be used independently in diagnosis, and the process thereof is complicated and high cost, since the signal probe is separately amplified after a target nucleic acid is amplified by conventional nucleic acid amplification such as PCR.

The asymmetric PCR method is derivative technology that selectively synthesizes a single strand DNA from a double strand DNA template which is efficiently used for nucleic acid sequencing. (Innis et al. Proc. Nalt. Sci. USA 85:9436, 1988, US 5,066,584) Besides, the asymmetric PCR method shows more efficient detection for adenovirus than the conventional symmetric PCR using the molecular beacon probe. (PoddarMol. Cell Probe 14:25, 2000) Addition to theses, the asymmetric PCR method is used to synthesize a template of FRET-type hybridization probe for detecting single nucleotide polymorphism of factor V gene. (Szilvasiet al. Clin. Chem. 38:727, 2005) The conventional symmetric PCR in which same amounts of forward and reverse primers are used is quiet probable that annealing between amplicons has a higher chance than annealing between the amplicon and the primer after some amount of amplicons are synthesized because the length of primers is much shorter than that of amplicons. Therefore there is more chance to anneal between amplicons than the amplicon and the primer causing lowering efficiency of amplification then, induces a plateau phase in which amplification is stopped eventually. (Rice et al. Prenatal Diagn. 22:1130, 2002) The asymmetric PCR method provides to solve the problem of annealing between amplicons by using different concentrations of the forward primer and the reverse primer to induce the amount of synthesized amplicons by the forward prime is not same as that of by the reverse primer. (Gyllenstenet al. Proc. Natl. Acad. Sci. USA 85:7652, 1988) When the limit amount of either of the primers is used all, then the excess amount of the primer induces linear amplification to improve amplification efficiency. Although efficiency of asymmetric PCR has better than that of symmetric PCR, the different is not significant because the high temperature of denaturation steps in PCR makes re-annealing between amplicons or the amplicon and the primer. (Gyllenstenet al. Methods Enzymol. 218:3, 1993) However in case of isothermal amplifications, the amplification efficiency different will be much bigger than that of PCR because the high temperature of denaturation steps is omitted in isothermal amplifications. In case, especially, the probe is used to anneal the synthesized amplicon either of the forward and reverse primers, the asymmetric method is very efficient. In other words, there is competitive annealing between amplicons versus between amplicon and the probe in symmetric amplification. Generally, the length of amplicons is much longer than that of a probe, thus annealing between amplicons is quiet probable than annealing between an amplicon and a probe. In the early stage of the amplification, annealing between the amplicon and the probe is quiet probable because the concentration of the probe is much higher than those of the amplicons. In the progression of the amplification, the concentration of the amplicons is getting higher thus annealing between amplicons is more probable and the signal amplification is stopped eventually. Therefore either of primers of which amplicon is annealed with the probe is used in an excess amount that induces increase of chance to anneal between the amplicon and the probe, thus chance of signal amplification by annealing between the amplicon and the probe is higher. As the result, mutual amplification efficiency is achieved.

One of the major problems of nucleic acid amplification methods including PCR is non-specific amplification. Non-specific amplification is caused to a false positive result and classified as a very critical problem to lower accuracy of diagnosis. Therefore, the most of primers are designed by cutting-edge technology such as bioinformatics employing computer simulation. Since a result of computer simulation is not always agreed to a real experiment result, the designed primer by bioinformatics cannot exclude non-specific amplification completely. In the amplification process, using an excess amount of primers is caused to primer-dimer as a result accuracy of nucleic acid amplification is limited. Especially, isothermal amplification methods compared to the PCR method are used longer primers that make the problem more serious. It is true that isothermal amplification methods compared to the PCR method are more difficult to achieve multiplex amplification in which more numbers of primers are required. However, this kind of problems can be solved by using an asymmetric amplification method. Because either of forward and reverse primers of which the concentration is significantly lower than the other that significantly prohibited the chance of the annealing between primers to reduce the primer-dimer result.

The present inventor had previous developed a method for detecting target nucleic acids by simultaneous isothermal amplification of nucleic acids and a signal probe employing same amounts of forward and reverse DNA-RNA-DNA hybrid primers. (Korean patent 10-0957057) However, this method has limitations to use a diagnosis method because it is difficult to detect below 100 CFUs per assay and tend to give false positive results by non-specific amplification. Addition to this, the previous method is too cumbersome in which the reaction mixture including the target DNA and the primer set is added and then the enzyme mixture is added to assay separately.

Accordingly, the present inventor has made extensive efforts in order to overcome the drawbacks described above and to improve a detection sensitivity of the target nucleic acid by accurately amplifying target nucleic acids, and at the same time, a method for detecting the amplification product, and as a result, confirmed that when an external primer set having a base sequence complementary to target nucleic acids and a DNA-RNA-DNA hybrid primer set having a base sequence partially complementary to target nucleic acids in which an asymmetric method composing of the concentration ratio of the forward and the reverse primers are differently used, it is possible to more efficiently amplify the target nucleic acids while minimizing non-specific amplification caused by annealing between primers at isothermal temperature. Also the present inventor confirmed that it is possible to simply and efficiently amplifying the target nucleic acid at isothermal temperature without an additional denaturation step according to the precedent isothermal LAMP amplification method, (Maruyameet al. Appl. Environ. Microbiol. 69:5023, 2003) thereby completing the present invention.

### SUMMARY OF INVENTION

The object of the present invention is to provide a method for amplifying a target nucleic acid and a signal probe at isothermal temperature simply and efficiently.

Another object of the present invention is to provide a method for detecting target nucleic acids, which comprises performing simultaneous isothermal amplification of target nucleic acids and probe signals.

Another objective of the present invention is to provide a method for detecting target nucleic acids, which comprises performing asymmetric amplification of target nucleic acids by employing a different concentration ratio of the forward and the reverse DNA-RNA-DNA hybrid primers and the DNA-RNA-DNA hybrid signal probe that has sequences complementary to the amplifying product of the excess amount primer used either of primers, the forward or the reverse DNA-RNA-DNA hybrid primers.

To achieve the above objects, the present invention provides a method for isothermal detection of target DNA, the method comprising the steps of:
(i) target DNA, (ii) an external primer set having a base sequence complementary to the target DNA, and (iii) a DNA-RNA-DNA hybrid primer set having a base sequence complementary to the target DNA at the 3'-terminal end DNA and non-complementary to the target DNA at the 5'-terminal end DNA-RNA, wherein the DNA-RNA-DNA hybrid primer set consists of 32∼66 bases in length, the both DNA regions of the DNA-RNA-DNA hybrid primer are 15∼30 bases in length, and the RNA region of the DNA-RNA-DNA hybrid primer is 1∼6 bases in length; and (iv) said DNA-RNA-DNA hybrid primer set having an excess amount either of the forward and reverse DNA-RNA-DNA hybrid primers uses to induce asymmetric amplification; and (v)adding an enzymatic reaction mixture solution containing RNase, DNA polymerase capable of performing strand displacement and a DNA-RNA-DNA hybrid signal probe having a base sequence complementary to the amplification product produced by said external primer set and said hybrid primer set, wherein the DNA-RNA-DNA hybrid signal probe consists of 18∼38 bases in length, the both DNA regions of the DNA-RNA-DNA hybrid probe are 8∼16 bases in length, and the RNA region of the DNA-RNA-DNA hybrid probe is 1∼6 bases in length, and then simultaneously amplifying said target DNA and said signal probe and detecting said target DNA at isothermal temperature.

Other features and aspects of the present invention will be apparent from the following detailed description and the appended claims.

### BRIEF DESCRIPTION OF DRAWINGS

FIG. 1 is a schematic figure of the method for isothermal amplification of target DNA according to the present invention.
FIG. 2 is a schematic figure of the method for isothermal amplification of target DNA and a signal probe according to the present invention.
FIG. 3 is a schematic figure of the difference between the method for symmetric isothermal amplification and the method for asymmetric isothermal amplification according to the present invention.
FIG. 4 is analysis results of detecting fluorescent signals (Delta Rn) by symmetric isothermal amplification and asymmetric isothermal amplification of *M. tuberculosis* according to the present invention, by means of real-time fluorescent detection.
FIG. 5 is analysis results of detecting fluorescent signals (Delta Rn) by symmetric isothermal amplification and asymmetric isothermal amplification of C. *trachomatis* according to the present invention, by means of real-time fluorescent detection.
FIG. 6 is analysis results of detecting fluorescent signals (Delta Rn) by symmetric isothermal amplification and asymmetric isothermal amplification of *N. gonorrhoease* according to the present invention, by means of real-time fluorescent detection.
FIG. 7 is analysis results of detecting fluorescent signals (Delta Rn) by symmetric isothermal amplification and asymmetric isothermal amplification of *L. monocytogenes* according to the present invention, by means of real-time fluorescent detection.
FIG. 8 is analysis results of detecting fluorescent signals (Delta Rn) by symmetric isothermal amplification and asymmetric isothermal amplification of *Salmonella* according to the present invention, by means of real-time fluorescent detection.

### DETAILED DESCRIPTION OF THE INVENTION,

### AND PREFERRED EMBODIMENTS

In one aspect, the present invention relates to a method for isothermal detection of target DNA, the method comprising the steps of: (i) target DNA, (ii) an external primer set having a base sequence complementary to the target DNA, and (iii) a DNA-RNA-DNA hybrid primer set having a base sequence complementary to the target DNA at the 3'-terminal end DNA and non-complementary to the target DNA at the 5'-terminal end DNA-RNA, and (iv) a DNA-RNA-DNA hybrid signal probe having a base sequence complementary to the amplification product produced by said external primer set and said hybrid primer set including an enzymatic reaction mixture solution containing RNase and DNA polymerase capable of performing strand displacement, and then simultaneously amplifying said target DNA and said signal probe and detecting said target DNA at isothermal temperature. The said DNA-RNA-DNA hybrid primer set having an excess amount either of primers, the forward or the reverse DNA-RNA-DNA hybrid primer is used to asymmetrically amplify a single strand nucleic acid.

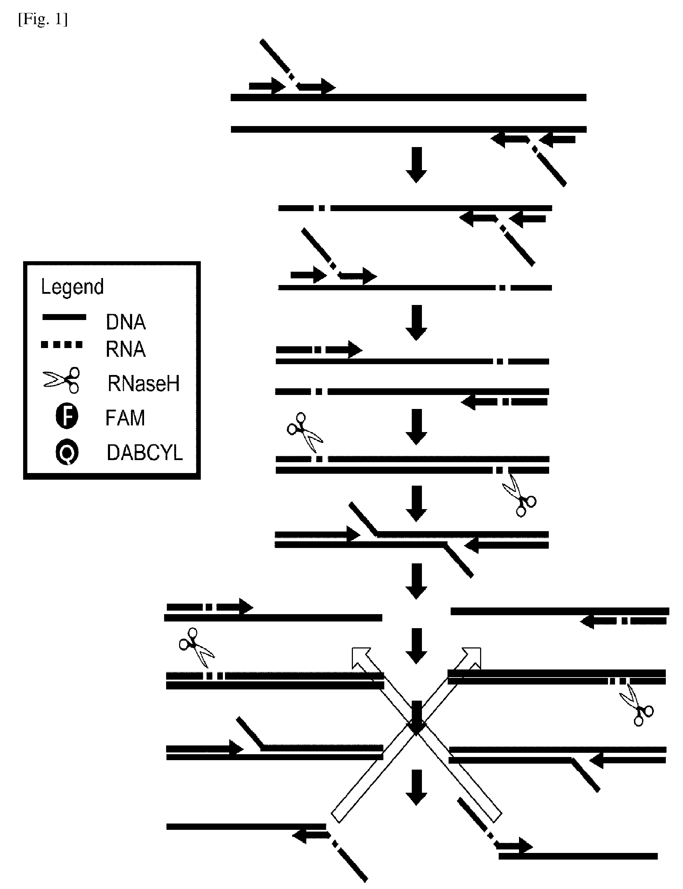

The isothermal amplification of target DNA according to the present invention is carried out in the following manner as shown in FIG. 1.

A mixture of target DNA to be amplified as a template in amplification, an external primer set, a DNA-RNA-DNA hybrid primer set, a DNA-RNA-DNA hybrid signal probe, and an enzymatic reaction mixture solution containing RNase and DNA polymerase is added thereto.

The external primer set and DNA-RNA-DNA hybrid primer set are then annealed to the target DNA in the reaction solution at the amplification temperature. Preferably, the external primer set comprises a sequence complementary to a sequence closer to both ends of the target DNA than the hybrid primer set, and the hybrid primer set comprises a sequence closer to the middle of the target DNA than the external primer set. In this case, the hybrid primer is annealed in the forward direction of DNA strand extension compared with the external primer. The annealed external primer and hybrid primer are extended using a DNA polymerase capable of performing strand displacement. As the external primer is extended along target DNA, DNA strand extended from the hybrid primer located in the forward direction of extension is separated from target DNA to result in a strand displacement. Finally, single stranded DNA amplification product extended from the hybrid primer and double stranded DNA amplification product extended from the external primer, respectively, are obtained.

The external primer set and the hybrid primer set are annealed using single stranded DNA amplification product as a template. The annealed external primer and hybrid primer are extended by a DNA polymerase capable of performing strand displacement, and as the external primer is extended along a single stranded DNA template, a DNA strand extended from the hybrid primer located in the forward direction of extension is separated from a single stranded DNA to result in strand displacement. Finally, the single stranded DNA amplification product extended from the hybrid primer and the double stranded DNA amplification product extended from the external primer are obtained. The external primer is extended to form a double stranded DNA and the extended DNA-RNA-DNA hybrid primer is separated by strand displacement to obtain a single stranded DNA. The DNA-RNA-DNA hybrid primer is annealed and extended using the amplified single stranded DNA as a template to obtain a double stranded DNA amplification product containing RNA. The RNA region of the double stranded DNA is digested by RNase H, and a single stranded DNA is obtained by strand displacement. Annealing, extension, strand displacement and RNA digestion process is repeated using the single stranded DNA as a template to amplify the target DNA (FIG. 1).

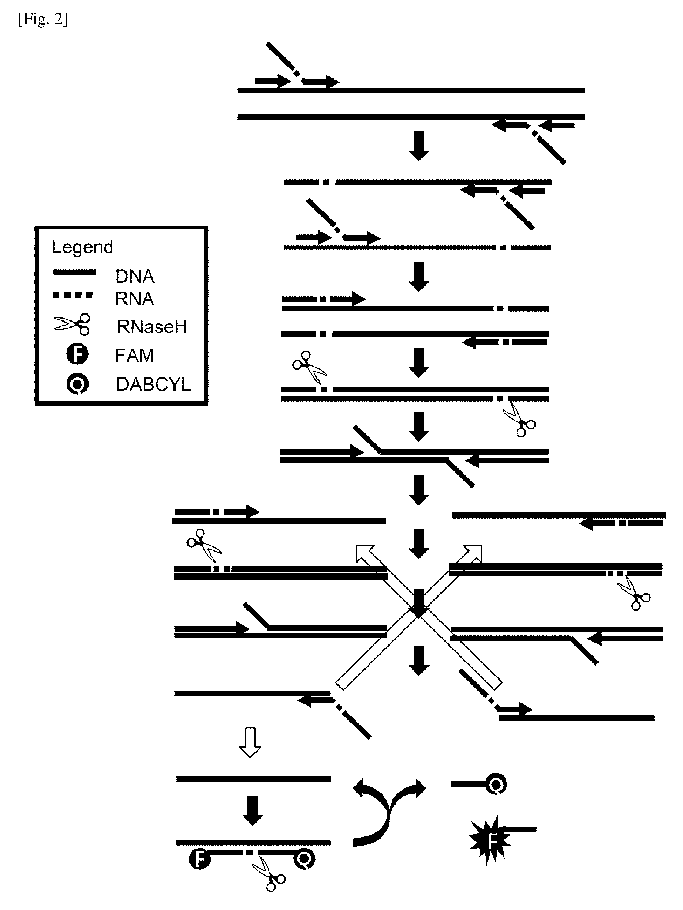

According to one embodiment of the present invention, amplification of a probe signal is simultaneously performed with isothermal amplification of the nucleic acids. After a target DNA amplified by isothermal amplification of the target DNA is annealed with a DNA-RNA-DNA hybrid signal probe to form a double stranded RNA/DNA hybrid, the RNA region of the DNA-RNA-DNA hybrid probe is digested by RNase H activity. Then, the digested signal probe is separated from the target DNA, followed by the binding of an intact DNA-RNA-DNA hybrid signal probe to be digested with RNase H and separated. The above described process is repeated to amplify the probe signal (FIG. 2).

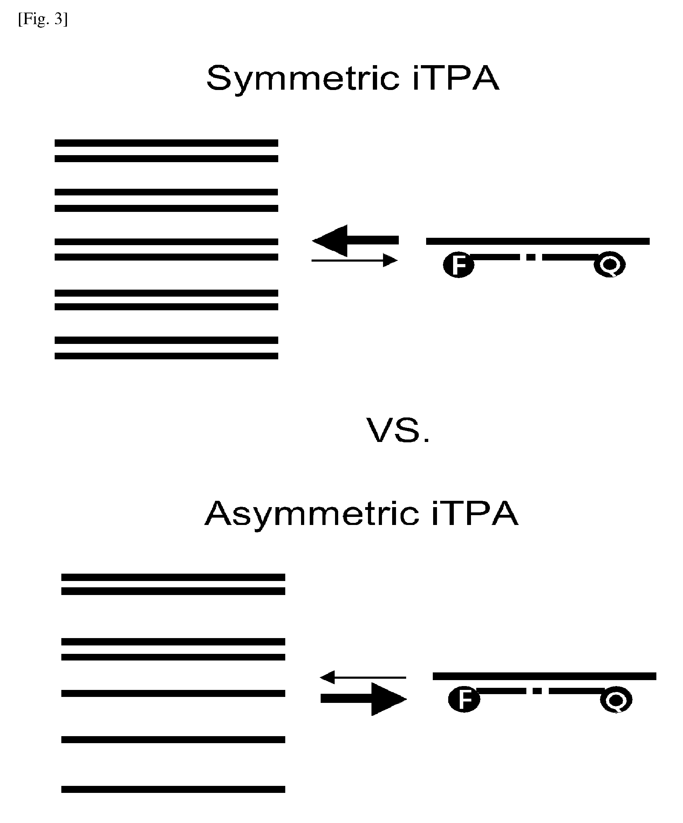

According to one embodiment of the present invention, the hybrid primers set has a different concentration ratio of the forward hybrid primer and the reverse hybrid primer and then an excess amount of the primer produces the excess amount of the single strand of amplicon which has a more chance to anneal with a complementary signal probe. As the result, asymmetric isothermal amplification has a higher chance to amplify the signal probe than symmetric isothermal amplification (FIG. 3). Especially, the efficiency of probe amplification in symmetric amplification is low since the length of amplicons is longer that of the probe in which annealing between amplicons is thermodynamically favorable. However, the excess amount of the single strand amplicon is annealed with the probe more efficiently to induce signal amplification in asymmetric amplification therefore the sensitivity of detecting target DNA is highly improved.

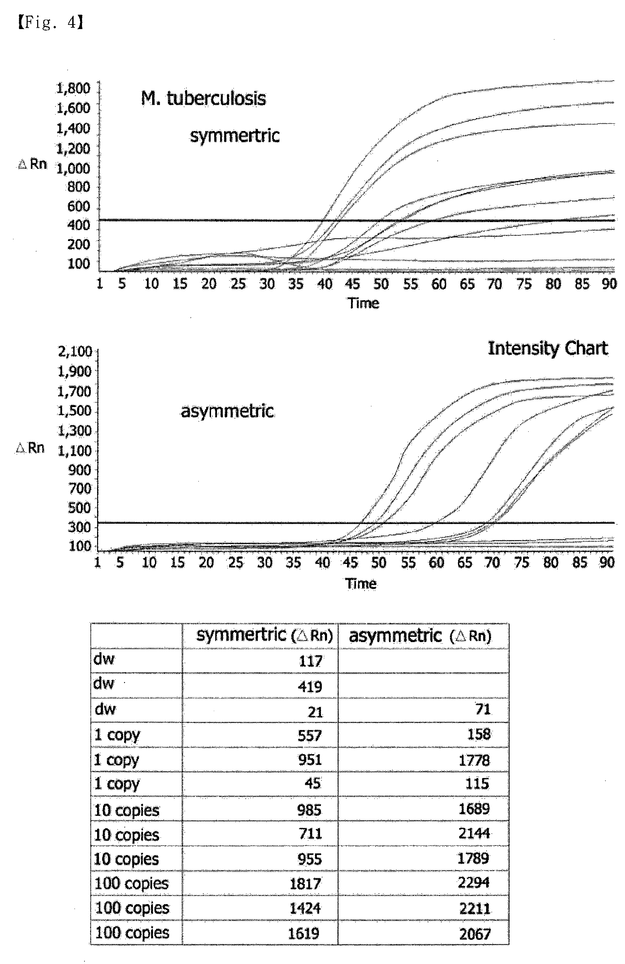

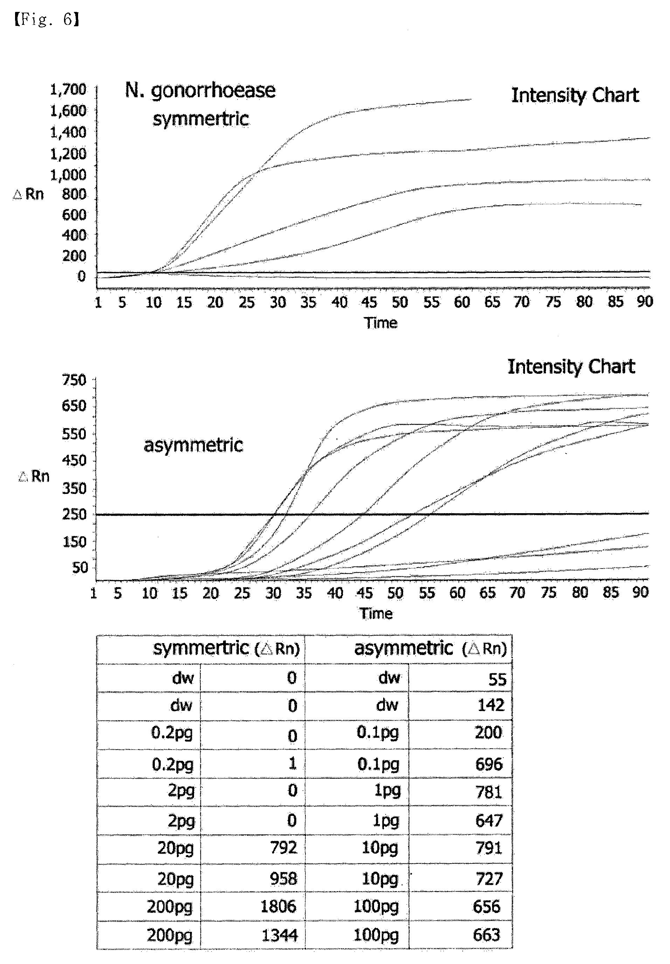

As shown in FIG.4 ∼ FIG. 6, the sensitivity of asymmetric isothermal amplification methods is improved compared to that of conventional symmetric isothermal amplification methods.

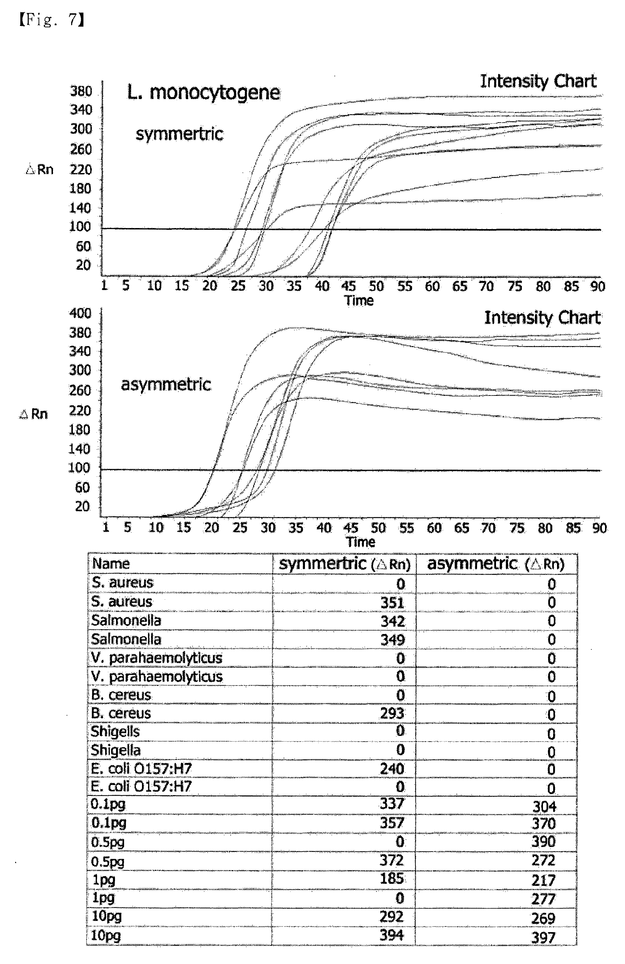

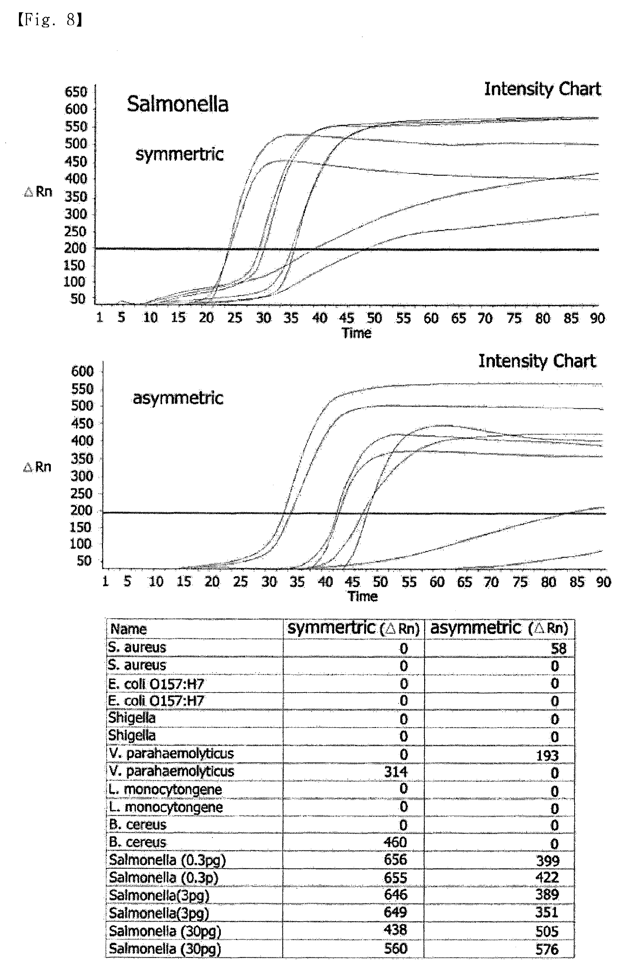

As shown in FIG.7 ∼ FIG. 8, the specificity of asymmetric isothermal amplification methods is improved compared to that of conventional symmetric isothermal amplification methods.

In the present invention, the external primer set can be any one selected from the group consisting of oligo DNA, oligo RNA, and hybrid oligo RNA/DNA. The external primer set is preferably complementary to the sequence of a target nucleic acid, and preferably has 15∼30 bases in length. A target DNA sequence complementary to the external primer is preferably a sequence neighboring a target DNA sequence complementary to a hybrid primer (base gap is 1∼60 bp) and the target DNA sequence complementary to the external primer is preferably a sequence closer to the 3'-end of the target nucleic acid than a target DNA sequence complementary to a hybrid primer.

The DNA-RNA-DNA hybrid primer set used in the present invention is non- complementary to a target DNA at the 5 '-end of DNA-RNA, and complementary to the target DNA at the 3'-end of DNA. The DNA-RNA-DNA hybrid primer preferably consists of 32∼66 bases in length, and preferably, the both DNA regions of the DNA-RNA-DNA hybrid primerare 15∼30 bases in length each and the RNA region of the DNA-RNA-DNA hybrid primer is 1∼6 bases in length.

In the present invention, a target DNA sequence complementary to a DNA-RNA-DNA hybrid primer preferably has a sequence closer to the 5'-end of a target DNA than a target DNA sequence complementary to an external primer, and a target DNA sequence complementary to a hybrid primer is preferably a sequence neighboring a target DNA sequence complementary to an external primer (base gap is 1∼60 bp).

The DNA polymerase used in the present invention is an enzyme that can extend a nucleic acid primer along a DNA template, and should be capable of displacing a nucleic acid strand from polynucleotide strands. DNA polymerase that can be used in the present invention is preferably a thermo-stable DNA polymerase with no exonuclease activity and examples thereof include Bst DNA polymerase, exo(-) vent DNA polymerase, exo(-) Deep vent DNA polymerase, exo(-) Pfu DNA polymerase, Bca DNA polymerase or phi29 DNA polymerase etc.

The RNase used in the present invention specifically digests the RNA strand of an RNA/DNA hybrid, and it is preferable not to degrade a single stranded RNA, and RNase H is preferably used.

The DNA-RNA-DNA hybrid signal probe used in the present invention is preferably an oligonucleotide having a sequence complementary to a nucleic acid amplification products amplified by the excess amount DNA-RNA-DNA hybrid primer used either of hybrid primers, the forward primer or the reverse primer, and the 5'-end and 3'-end of the DNA-RNA-DNA hybrid signal probe consist of oligo DNA and the middle thereof consists of oligo RNA.

Preferably, the DNA-RNA-DNA hybrid signal probe consists of 18∼38 bases in length, and preferably, the both DNA regions of the DNA-RNA-DNA hybrid signal probeare8∼16 bases in length each and the RNA region of the DNA-RNA-DNA hybrid primer is 1∼6 bases in length.

In the present invention, the DNA-RNA-DNA hybrid signal probe is preferably labeled with a marker at an end, and the markers are fluorescence and quencher.

In the present invention, the concentration ratio of the forward DNA-RNA-DNA hybrid primer and the reverse DNA-RNA-DNA primer is preferably from 1:5 to 1:20 or from5:1 to 20:1 and more preferably 1:10 or 10:1. The amplified product of the excessively used hybrid primer is complementary to the hybrid signal probe.

In the present invention, the isothermal amplification reaction is preferably performed at a temperature at which the inventive primer and probe can be annealed to the DNA template, and the activity of an enzyme used is not substantially inhibited. In the present invention, the amplification temperature is preferably 30-75°C, more preferably 37-70°C, most preferably 55∼65 °C.

Moreover, the inventive method for isothermal amplification of nucleic acids has high specificity, since it uses an additional external primer compared with conventional methods in which a single RNA-DNA hybrid primer is used (US 6,251,639). Besides, it is possible to significantly improve amplification efficiency by exponential amplification using an inner primer substituted by an external primer as a new template. Moreover, the conventional method uses a separate blocker for blocking amplification or a template-switch oligonucleotide (TSO) to amplify a specific region upon amplification of target base sequences using a single RNA- DNA hybrid primer, on the contrary, the inventive method has an advantage in that only a desired region can be clearly amplified using a forward/reverse primer pair without using a separate blocker or TSO.

Also, the inventive method has an advantage in that the DNA-RNA-DNA hybrid signal probe is bound and separated using an amplified DNA as a template, and amplifies the signal probe. Therefore it can simultaneously amplify nucleic acids and a signal probe completed in a single-tube by repeating a process compared to known prior methods. US patent 5,824,517 discloses an isothermal amplification using an external primer set and a DNA-RNA-DNA hybrid primer set, but it does not use of a DNA-RNA-DNA hybrid signal probe. Also US patent application 2005/0214809 discloses the use of a DNA-RNA-DNA hybrid signal probe which is used for signal probe amplification only.

The invention is an improved isothermal amplification method in that the hybrid primers set has a different concentration ratio of the forward hybrid primer and the reverse hybrid primer compared to the conventional symmetric isothermal amplification in which the same concentration ratio of hybrid primers is used. Using an excess amount of one type hybrid primer produces an excess amount of one type single strand amplicon that has a more chance to anneal with a complementary signal probe. Thus asymmetric isothermal amplification amplifies the signal probe more efficiently than symmetric isothermal amplification and as the result the sensitivity and the specificity of the nucleic acid detection are improved.

The inventive method also has an advantage in that it does not need to consider problems occurring when reaction activity of RNase is higher than primer extension activity of DNA polymerase in the conventional method, because the 5'-end of DNA-RNA region of the DNA-RNA-DNA hybrid primer used in the present invention, has a sequence non-complementary to a template.

In the inventive isothermal amplification of nucleic acids, a newly synthesized amplification product is used as a new template after a first primer extension and strand displacement reaction and the 5'-end DNA-RNA region non-complementary to the template acts as a template complementary to a primer to increase the annealing temperature for the primer, thus improving amplification efficiency, as well as, preventing primer-dimer formation to enhance the purity of the amplification product.

The method for isothermal amplification of nucleic acids according to the present invention requires about 1 hr for complete amplification, starting from DNA extraction in a sample. If DNA extraction was already completed, it requires about 40 minutes, thereby making it is possible to perform rapid amplification.

In another aspect, the present invention provides a method for detecting target DNA, the method comprising the steps of: : (i) target DNA, (ii) an external primer set having a base sequence complementary to the target DNA, and (iii) a DNA-RNA-DNA hybrid primer set having a base sequence complementary to the target DNA at the 3'-terminal end DNA and non-complementary to the target DNA at the 5'-terminal end DNA-RNA, and (iv) a DNA-RNA-DNA hybrid signal probe having a base sequence complementary to the amplification product produced by said external primer set and said hybrid primer set including an enzymatic reaction mixture solution containing RNase and DNA polymerase capable of performing strand displacement, and then simultaneously amplifying said target DNA and said signal probe and detecting said target DNA at isothermal temperature. The said DNA-RNA-DNA hybrid primer set having an excess amount either of primers, the forward or the reverse DNA-RNA-DNA hybrid primer is used to asymmetrically amplify a single strand nucleic acid.

The signal probe amplified according to the method of the present invention can be detected using a fluorescent detector in a reaction tube without any additional post-amplification process. In this case, the DNA-RNA-DNA hybrid probe is preferably end-labeled with fluorescence and quencher that FRET (fluorescence resonance energy transfer) type signal probe in which fluorescent signal is not emitted until fluorescence and quencher are separated by cleavaging of the signal probe.

The inventive isothermal amplification and detection method of nucleic acids can amplify in a rapid and simple manner since it employs one-step method in which the reaction is carried out at a constant temperature, and thus it does not require any thermal cycling device due to isothermal amplification of the target nucleic acids and the signal probe. Additionally, the method exactly amplifies specific the target nucleic acid region by using two pairs of primers as well as amplifies the signal probe, and asymmetrically amplifies the target nucleic acid by using a different concentration ratio of a forward hybrid primer and a reverse hybrid primer to give a higher chance to anneal between the amplified product and the signal probe compared to the conventional symmetrical amplification method, As the result, the sensitivity and specificity of the nucleic acid detection are improved.

The inventive isothermal amplification and detection method of the nucleic acid is carried out in one tube and thus it is possible to treat in large quantities for real-time detection of nucleic acids. Such advantage can minimize the risk of an additional reaction by contamination which limits a wide use of amplification technique.

### Examples

Hereinafter, the present invention will be described in more detail by examples. However, it is obvious to a person skilled in the art that these examples are for illustrative purposes only and are not construed to limit the scope of the present invention.

### Example 1: Isothermal amplification of M. tuberculosisDNA

The commercially available *Mycobacterium tuberculosis* genomic DNA (Vircell, Spain) DNA was used as a template by quantified serial dilutions.

External primers (SEQ ID NO: land SEQ ID NO: 2) were designed such that they comprise sequences complementary to *Mycobacterium tuberculosis* IS6110. SEQ ID NO: 1 is forward and sense and SEQ ID NO:2 is reverse and anti-sense.
SEQ ID NO 1: 5'-CGATCGAGCAAGCCA-3'
SEQ ID NO2: 5'-CGAGCCGCTCGCTGA-3'

DNA-RNA-DNA hybrid primers (SEQ ID NO: 3 and SEQ ID NO: 4) were designed such that the 5'-end of oligo DNA-RNA region thereof has a sequence non-complementary to *Mycobacterium tuberculosis* IS6110, and the 3'-end of oligo DNA region thereof has a sequence complementary to *Mycobacterium tuberculosis* IS6110.SEQ ID NO: 3 is forward and sense and SEQ ID NO: 4 is reverse and anti-sense.(oligo RNA region is underlined).
SEQ ID NO: 3:5'-CGATGACTGACTATACAAGAGGAAAGGCGTACTCGACCYGA-3'
SEQ ID NO: 4: 5'-CATTCCAGTTAAGCTAGCAGGTACTGAGATCCCCT-3'

A DNA-RNA-DNA hybrid signal probe (SEQ ID NO: 5) for performing signal amplification, has a base sequence complementary to DNA amplified by the above primer set, and is labeled with a FAM dye and a DABCYL quencher at the 5'-end and the 3'-end thereof, respectively (oligo RNA region is underlined):
SEQ ID NO: 5: 5'-FAM-ATCCGTAUGGTGGATAACGTCTTTCA-DABCYL-3'

In order to amplify target nucleic acids using the external primer set and the hybrid primer set, a reaction mix and an enzyme mixture were prepared. The reaction mixture containing the external primer set, the hybrid primer set and target DNA was prepared. 10mM Tris-HCl (pH 8,5) bluffer, 10 mM (NH₄)₂SO₄, 10mM MgSO4, 50mM KCl, 1.6mM dNTP (Fermentas), 2.6mM DTT, 0.1µg BSA, 0.6mMspermine, 100mM trehalose, 0.11µM of external primer set (IDT), and 1.1µtM of hybrid primer set (in case of asymmetric amplification 2.2µMforward primer (sense) and 0.22µM reverse hybrid primer (anti-sense))was prepare the reaction mixture. The enzyme mixture containing 1 unit Bst polymerase (NEB), 6 units RNase H (Epicentre), 6 units RNase inhibitor, and 50 nM hybrid signal probe (IDT) was prepared.

In a 0.2mL PCR tube, 5µL of the above reaction mixture, 5µL of the above enzyme mixture, and 10µL of serially diluted template DNA were added at room temperature and performed isothermal amplification at 61°C for 1.5 hr. Distilled water was used as a negative control. The real time isothermal fluorescent detector (Scinco, Korea) was set for reaction temperature to 61°C and for acquiring fluorescent signal every 1 min.

As a result, as shown in FIG. 4, it was confirmed that the sensitivity of asymmetric isothermal amplification is better than that of the symmetric isothermal amplification.

### Example 2: Isothermal amplification of C. trachomatisDNA

*Chlamydia trachomatis*genomic DNA was used as a template. Genomic DNA was extracted from the *Chlamydia trachomatis*strain (ATCC Cat. No. VR0887) using the G-spin™ Genomic DNA extraction Kit (iNtRON Biotechnology, Cat. No.17121), then subjected to amplification. For the genomic DNA extraction, 500µL of the bacterial suspension was centrifuged at 13,000 rpm for 1 min and the supernatant was removed then, 500µL of PBS (pH 7.2) was added thereto, followed by centrifuging to remove supernatant. Then, cell pellets were suspended by adding 300µL of G-buffer solution containing RNase A and Proteinase K, and left to stand at 65°C for 15min, then 250µL of binding buffer solution was added thereto to mix thoroughly, followed by binding DNA to a spin column. After that, 500µL of washing buffer A was added to the spin column and centrifuged at 13,000 rpm for 1 min to wash, and 500 µL of washing buffer B was added to the spin column to centrifuge, then the column was moved to a 1.5mL microcentrifuge tube, followed by adding 50µL of elution buffer to centrifuge for 1 min, thus obtaining 15.8ng/mL genomic DNA. The obtained genomic DNA was diluted in a given ratio and used as a template of isothermal amplification reaction.

External primers (SEQ ID NO: 6 and SEQ ID NO: 7) were designed such that they comprise sequences complementary to *Chlamydia trachomatis* cryptic plasmid DNA.SEQ ID NO: 6 is forward and sense and SEQ ID NO: 7 is reverse and anti-sense.
SEQ ID NO: 6: 5'-TAAACATGAAAACTCGTTCCG-3'
SEQ ID NO: 7: 5'-TTTTATGATGAGAACACTTAAACTCA-3'

DNA-RNA-DNA hybrid primers (SEQ ID NO: 8 and SEQ ID NO: 9) were designed such that the 5'-end of oligo DNA-RNA region thereof has a sequence non-complementary to *Chlamydia trachomatis* cryptic plasmid DNA, and the 3'-end of oligo DNA region thereof has a sequence complementary to *Chlamydia trachomatis* cryptic plasmid DNA.SEQ ID NO: 8 is forward and sense and SEQ ID NO: 9 is reverse and anti-sense. (oligo RNA region is underlined).
SEQ ID NO: 8:5'-ACCGCATCGAATCGATGTAAAATAGAAAATCGCATGCAAGATA-3'
SEQ ID NO: 9: 5'-CGATTCCGCTCCAGACTTAAAAAGCTGCCTCAGAATATACTCAG -3'

DNA-RNA-DNA hybrid signal probe (SEQ ID NO: 10) for performing signal amplification, has a base sequence complementary to DNA amplified by the above primer set, and is labeled with a FAM dye and BHQ1 quencher at the 5'-end and the 3'-end thereof, respectively (oligo RNA region is underlined):
SEQ ID NO: 10: 5'-FAM-GGTAAAGCTCUGAUAUTTGAAGACTCT-BHQ1-3'

In order to amplify target nucleic acids using the external primer set and the hybrid primer set, a reaction mix and an enzyme mixture were prepared. The reaction mixture containing the external primer set, the hybrid primer set and target DNA was prepared. 10mM Tris-HCl (pH 8,5) buffer, 10mM (NH₄)₂SO₄, 20mM MgSO4, 10mM KCl, 1mM dNTP (Fermentas), 1mMDTT, 0.1µg BSA, 0.15mMspermine, 20mM trehalose, 0.2µM of external primer set (IDT), and 1µM of hybrid primer set (in case of asymmetric amplification 0.4µM forward primer (sense) and 2µM reverse hybrid primer (anti-sense)) was prepare the reaction mixture. The enzyme mixture containing 3 unit Bst polymerase (NEB), 8 units RNase H (Epicentre), 6 units RNase inhibitor, and 50 nM hybrid signal probe (IDT) was prepared.

In a 0.2 mL PCR tube, 5 µL of the above reaction mixture, 5 µL of the above enzyme mixture, and 10 µL of serially diluted template DNA were added at room temperature and performed isothermal amplification at 61°C for 1.5 hr. Distilled water was used as a negative control. The real time isothermal fluorescent detector (Scinco, Korea) was set for reaction temperature to 61°C and for acquiring fluorescent signal every 1 min.

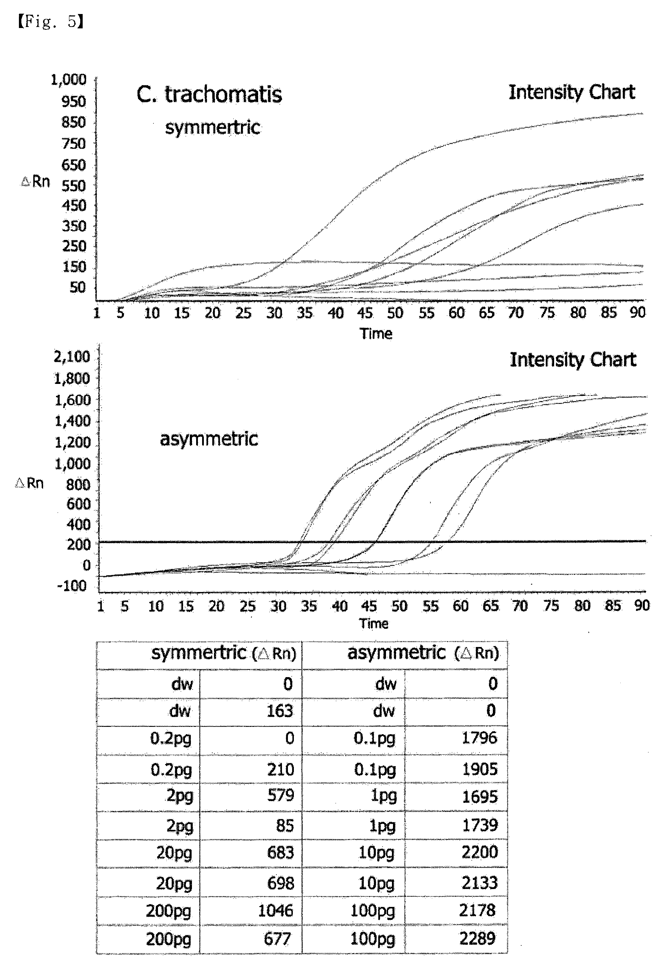

As a result, as shown in FIG. 5, it was confirmed that the sensitivity of asymmetric isothermal amplification is better than that of the symmetric isothermal amplification.

### Example 3: Isothermal amplification of N. gonorrhoeaseDNA

*Neisseria gonorrhoease* genomic DNA was used as a template. Genomic DNA was extracted from the *Neisseria gonorrhoease* strain (ATCC Cat. No. 49226) using the G-spin™ Genomic DNA extraction Kit (iNtRON Biotechnology, Cat. No.17121), then subjected to amplification. For the genomic DNA extraction, 500µL of the bacterial suspension was centrifuged at 13,000 rpm for 1 min and the supernatant was removed then, 500µL of PBS (pH 7.2) was added thereto, followed by centrifuging to remove supernatant. Then, cell pellets were suspended by adding 300µL of G-buffer solution containing RNase A and Proteinase K, and left to stand at 65°C for 15min, then 250µL of binding buffer solution was added thereto to mix thoroughly, followed by binding DNA to a spin column. After that, 500µL of washing buffer A was added to the spin column and centrifuged at 13,000 rpm for 1 min to wash, and 500 µL of washing buffer B was added to the spin column to centrifuge, then the column was moved to a 1.5mL microcentrifuge tube, followed by adding 50µL of elution buffer to centrifuge for 1 min, thus obtaining 4.7ng/mL genomic DNA. The obtained genomic DNA was diluted in a given ratio and used as a template of isothermal amplification reaction.

External primers (SEQ ID NO: 11 and SEQ ID NO: 12) were designed such that they comprise sequences complementary to *Neisseria gonorrhoeaseopa* gene DNA.SEQ ID NO: 11 is forward and sense and SEQ ID NO: 12 is reverse and anti-sense.
SEQ ID NO: 11: 5'-TCATCCGCCATATTGTG-3'
SEQ ID NO: 12: 5'-TTTCGGCTCCTTATTCGGTTT-3'

DNA-RNA-DNA hybrid primers (SEQ ID NO: 13 and SEQ ID NO: 14) were designed such that the 5'-end of oligo DNA-RNA region thereof has a sequence non-complementary to *Neisseria gonorrhoeaseopa* gene DNA, and the 3'-end of oligo DNA region thereof has a sequence complementary to *Neisseria gonorrhoeaseopa* gene DNA.SEQ ID NO: 13 is forward and sense and SEQ ID NO: 14 is reverse and anti-sense. (oligo RNA region is underlined).
SEQ ID NO: 13:5'-GCATAGCTCAAGTATATGGCACATTGAAACACCGCCCGGAA-3'
SEQ ID NO: 14: 5'-CTATCAGTGAAGCTAACGACCGGTTAAAAAAATTTTCACTG-3'

DNA-RNA-DNA hybrid signal probe (SEQ ID NO: 15) for performing signal amplification, has a base sequence complementary to DNA amplified by the above primer set, and is labeled with a FAM dye and DABCYL quencher at the 5'-end and the 3'-end thereof, respectively (oligo RNA region is underlined):
SEQ ID NO: 15: 5'-FAM-ATGTTGAAGGACGGATTATATCGG-DABCYL-3'

In order to amplify target nucleic acids using the external primer set and the hybrid primer set, a reaction mix and an enzyme mixture were prepared. The reaction mixture containing the external primer set, the hybrid primer set and target DNA was prepared. 10mM Tris-HCl (pH 8,5) buffer, 10mM (NH₄)₂SO₄, 10mM MgSO4, 10mM KCl, 1.6mM dNTP (Fermentas), 5mM DTT, 0.1µg BSA, 0.4mMspermine, 20mM trehalose, 0.1µM of external primer set (IDT), and 1µM of hybrid primer set (in case of asymmetric amplification 4.4µM forward primer (sense) and 0.44µM reverse hybrid primer (anti-sense)) was prepare the reaction mixture. The enzyme mixture containing 5 unit Bstpolymerase (NEB), 5 units RNase H (Epicentre), 6 units RNase inhibitor, and 30 nM hybrid signal probe (IDT) was prepared.

In a 0.2 mL PCR tube, 5 µL of the above reaction mixture, 5 µL of the above enzyme mixture, and 10 µL of serially diluted template DNA were added at room temperature and performed isothermal amplification at 61°C for 1.5 hr. Distilled water was used as a negative control. The real time isothermal fluorescent detector (Scinco, Korea) was set for reaction temperature to 61°C and for acquiring fluorescent signal every 1 min.

As a result, as shown in FIG. 6, it was confirmed that the sensitivity of asymmetric isothermal amplification is better than that of the symmetric isothermal amplification.

### Example 4: Isothermal amplification of L. monocytogenesDNA

*Listeria monocytogenes* genomic DNA was used as a template. Genomic DNA was extracted from the *Listeria monocytogenes* strain (ATCC Cat. No. 35152) using the G-spin™ Genomic DNA extraction Kit (iNtRON Biotechnology, Cat. No.17121), then subjected to amplification. For the genomic DNA extraction, 500µL of the bacterial suspension was centrifuged at 13,000 rpm for 1 min and the supernatant was removed then, 500µL of PBS (pH 7.2) was added thereto, followed by centrifuging to remove supernatant. Then, cell pellets were suspended by adding 300µL of G-buffer solution containing RNase A and Proteinase K, and left to stand at 65°C for 15min, then 250µL of binding buffer solution was added thereto to mix thoroughly, followed by binding DNA to a spin column. After that, 500µL of washing buffer A was added to the spin column and centrifuged at 13,000 rpm for 1 min to wash, and 500 µL of washing buffer B was added to the spin column to centrifuge, then the column was moved to a 1.5mL microcentrifuge tube, followed by adding 50µL of elution buffer to centrifuge for 1 min, thus obtaining 8.4 ng/mL genomic DNA. The obtained genomic DNA was diluted in a given ratio and used as a template of isothermal amplification reaction.

External primers (SEQ ID NO: 16 and SEQ ID NO: 17) were designed such that they comprise sequences complementary to *Listeria monocytogenesactA* gene DNA.SEQ ID NO: 16 is forward and sense and SEQ ID NO: 17 is reverse and anti-sense.
SEQ ID NO: 16: 5'-TGCGTGCCATGATGGTAGTTTT-3'
SEQ ID NO: 17: 5'-CTTGGCTGCTCTTCTGTTTT-3'

DNA-RNA-DNA hybrid primers (SEQ ID NO: 18 and SEQ ID NO: 19) were designed such that the 5'-end of oligo DNA-RNA region thereof has a sequence non-complementary to *Listeria monocytogenesactA* gene DNA, and the 3'-end of oligo DNA region thereof has a sequence complementary to *Listeria monocytogenesactA* gene DNA.SEQ ID NO: 18 is forward and sense and SEQ ID NO: 19 is reverse and anti-sense. (oligo RNA region is underlined).
SEQ ID NO: 18:5'-CATCACCAACAAGAAGTAGAUUATGCATTACGATTAACCCCGAC-3'
SEQ ID NO: 19: 5'-CTGTCATTCATAGTCTCGTCUACUTCTTCCCATTCATCTGTGTTCA-3'

DNA-RNA-DNA hybrid signal probe (SEQ ID NO: 20) for performing signal amplification, has a base sequence complementary to DNA amplified by the above primer set, and is labeled with a FAM dye and BHQ1 quencher at the 5'-end and the 3'-end thereof, respectively (oligo RNA region is underlined):
SEQ ID NO: 20: 5'-FAM-ATTTGCAGCGACAGATAGCGAAGA-BHQ1-3'

In order to amplify target nucleic acids using the external primer set and the hybrid primer set, a reaction mix and an enzyme mixture were prepared. The reaction mixture containing the external primer set, the hybrid primer set and target DNA was prepared. 10mM Tris-HCl (pH 8,5) buffer, 10mM (NH₄)₂SO₄, 12mM MgSO4, 10mM KCl, 1.8mM dNTP (Fermentas), 6mM DTT, 0.1µg BSA, 0.8mMspermine, 0.1µM of external primer set (IDT) (in case of asymmetric amplification 0.01µM forward primer (sense), and 0.1µM reverse primer (anti-sense), and 1µM of hybrid primer set (in case of asymmetric amplification, 0.1µM forward primer (sense) and 1µM reverse hybrid primer (anti-sense)) was prepare the reaction mixture. The enzyme mixture containing 3 unit Bst polymerase (NEB), 6 units RNase H (Epicentre), 6 units RNase inhibitor, and 15nM hybrid signal probe (IDT) was prepared.

In a 0.2 mL PCR tube, 5 µL of the above reaction mixture, 5 µL of the above enzyme mixture, and 10 µL of serially diluted template DNA were added at room temperature and performed isothermal amplification at 61°C for 1.5 hr. Distilled water was used as a negative control. For specificity tests, the genomic DNAs of *Staphylococcus aureus* (Korean collection type culture (KCTC) 1927), *Salmonella enterica* (KCTC 1925), *Vibrio parahaemolyticus* (ATCC 17802), *Bacillus cereus* (ATCC 49953), *Shigellaflexneri* (KCTC 2517), and *Escherichia coli O157:H7*ATCC 35150) were extracted as the same method for extracting genomic DNA of *Listeria monocytogenes* and used. The real time isothermal fluorescent detector (Scinco, Korea) was set for reaction temperature to 61°C and for acquiring fluorescent signal every 1 min.

As a result, as shown in FIG. 7, it was confirmed that the specificity of asymmetric isothermal amplification is better than that of the symmetric isothermal amplification.

### Example 5: Isothermal amplification of SalmonellaDNA

*Salmonella enterica* genomic DNA was used as a template. Genomic DNA was extracted from the *Salmonella enterica* strain (KCTC 1925) using the G-spin™ Genomic DNA extraction Kit (iNtRON Biotechnology, Cat. No.17121), then subjected to amplification. For the genomic DNA extraction, 500µL of the bacterial suspension was centrifuged at 13,000 rpm for 1 min and the supernatant was removed then, 500µL of PBS (pH 7.2) was added thereto, followed by centrifuging to remove supernatant. Then, cell pellets were suspended by adding 300µL of G-buffer solution containing RNase A and Proteinase K, and left to stand at 65°C for 15min, then 250µL of binding buffer solution was added thereto to mix thoroughly, followed by binding DNA to a spin column. After that, 500µL of washing buffer A was added to the spin column and centrifuged at 13,000 rpm for 1 min to wash, and 500 µL of washing buffer B was added to the spin column to centrifuge, then the column was moved to a 1.5mL microcentrifuge tube, followed by adding 50µL of elution buffer to centrifuge for 1 min, thus obtaining 6.7 ng/mL genomic DNA. The obtained genomic DNA was diluted in a given ratio and used as a template of isothermal amplification reaction.

External primers (SEQ ID NO: 21 and SEQ ID NO: 22) were designed such that they comprise sequences complementary to *Salmonella entericainvA* gene DNA.SEQ ID NO: 21 is forward and sense and SEQ ID NO: 22 is reverse and anti-sense.
SEQ ID NO: 21: 5'-CTGATTCTGGTACTAATGGTGATGATC-3'
SEQ ID NO: 22: 5'-CTGAGGATTCTGTCAATGTAGAACGA-3'

DNA-RNA-DNA hybrid primers (SEQ ID NO: 23 and SEQ ID NO: 24) were designed such that the 5'-end of oligo DNA-RNA region thereof has a sequence non-complementary to *Salmonella entericainvA* gene DNA, and the 3'-end of oligo DNA region thereof has a sequence complementary to *Salmonella entericainvA* gene DNA.SEQ ID NO: 23 is forward and sense and SEQ ID NO: 24 is reverse and anti-sense. (oligo RNA region is underlined).
SEQ ID NO: 23:5'-ACCTCAGACATCCAGGAGAGTTCGTCATTCCATTACC-3'
SEQ ID NO: 24: 5'-ATCGCAGATTAGGCTCAGAGAACACCAATATCGCCAGTA-3'

DNA-RNA-DNA hybrid signal probe (SEQ ID NO: 25) for performing signal amplification, has a base sequence complementary to DNA amplified by the above primer set, and is labeled with a FAM dye and BHQ1 quencher at the 5'-end and the 3'-end thereof, respectively (oligo RNA region is underlined):
SEQ ID NO: 25: 5'-FAM-TCAGTGCGATCAGGAAATCAACCAGATA-BHQ1-3'

In order to amplify target nucleic acids using the external primer set and the hybrid primer set, a reaction mix and an enzyme mixture were prepared. The reaction mixture containing the external primer set, the hybrid primer set and target DNA was prepared. 10mM Tris-HCl (pH 8,5) buffer, 10mM (NH₄)₂SO₄, 12mM MgSO4, 10mM KCl, 1.8mM dNTP (Fermentas), 6mM DTT, 0.1µg BSA, 0.75mMspermine, 0.075µM of external primer set (IDT) (in case of asymmetric amplification 0.075µM forward primer (sense), and 0.075µM reverse primer (anti-sense), and 1.125µM of hybrid primer set (in case of asymmetric amplification, 2.25µM forward primer (sense) and 0.225µM reverse hybrid primer (anti-sense)) was prepare the reaction mixture. The enzyme mixture containing 3 unit Bst polymerase (NEB), 6 units RNase H (Epicentre), 6 units RNase inhibitor, and 25 nM hybrid signal probe (IDT) was prepared.

In a 0.2 mL PCR tube, 5 µL of the above reaction mixture, 5 µL of the above enzyme mixture, and 10 µL of serially diluted template DNA were added at room temperature and performed isothermal amplification at 61°C for 1.5 hr. Distilled water was used as a negative control. For specificity tests, the genomic DNAs of *Staphylococcus aureus* (Korean collection type culture (KCTC) 1927), *Listeria monocytogenes* (ATCC 5152), *Vibrio parahaemolyticus* (ATCC 17802), *Bacillus cereus* (ATCC 49953), *Shigellaflexneri* (KCTC 2517), and *Escherichia coli O157:H7* ATCC 35150) were extracted as the same method for extracting genomic DNA of *Listeria monocytogenes* and used. The real time isothermal fluorescent detector (Scinco, Korea) was set for reaction temperature to 61°C and for acquiring fluorescent signal every 1 min.

As a result, as shown in FIG. 8, it was confirmed that the specificity of asymmetric isothermal amplification is better than that of the symmetric isothermal amplification.

### INDUSTRIAL APPLICABILITY

As described above in detail, the present invention provides a method for asymmetrically amplifying target nucleic acids rapidly and exactly at isothermal temperature, and a method for detecting nucleic acids, which comprises simultaneously performing amplifications of target nucleic acids and a signal probe at isothermal temperature. The method according to the present invention can be used to amplify target nucleic acids in a sample, rapid and exact manner without the risk of contamination compared to the conventional methods such as PCR, and it can simultaneously and asymmetrically amplify target nucleic acid and a signal probe, so that it can be applied to various genome projects, detection and identification of a pathogen, detection of gene modification producing a predetermined phenotype, detection of hereditary diseases or determination of sensibility to diseases, and estimation of gene expression. Thus, it is useful for molecular biological studies and disease diagnosis.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A method for isothermal detection of target DNA, comprising:
(i) preparing target DNA;
(ii) preparing an external primer set having a base sequence complementary to the target DNA;
(iii) preparing a DNA-RNA-DNA hybrid primer set having a base sequence complementary to the target DNA at the 3'-terminal end and non-complementary to the target DNA at the 5'-terminal end;
(iv) preparing a DNA-RNA-DNA hybrid primer set having an excess amount either of the forward or reverse DNA-RNA-DNA hybrid primers uses to induce asymmetric amplification; and
(v) adding an enzymatic reaction mixture solution containing RNase, DNA polymerase capable of performing strand displacement and a DNA-RNA-DNA hybrid signal probe having a base sequence complementary to the amplification product produced by said external primer set and said hybrid primer set, and then simultaneously amplifying said target DNA and said signal probe and detecting said target DNA at isothermal temperature.

2. The method for isothermal detection of target DNA according to claim 1, wherein the external primer set is any one selected from the group consisting of oligo DNA, oligo RNA, and hybrid oligo RNA/DNA.

3. The method for isothermal detection of target DNA according to claim 1, wherein the DNA-RNA-DNA hybrid primer set is non-complementary to a target DNA at the 5'-end of DNA-RNA, and complementary to the target DNA at the 3'-end of DNA.

4. The method for isothermal detection of target DNA according to claim 1, wherein the DNA-RNA-DNA hybrid primer set is using an excess amount either of the forward or reverse DNA-RNA-DNA hybrid primers.

5. The method for isothermal detection of target DNA according to claim 4, wherein the concentration ratio of the forward and the reverse DNA-RNA-DNA hybrid primer is from 1:5 to 1:20 and vice versa.

6. The method for isothermal detection of target DNA according to claim 1, wherein the DNA-RNA-DNA hybrid signal probe is complementary to the amplified product by the excess amount of the DNA-RNA-DNA hybrid primer.

7. The method for isothermal detection of target DNA according to claim 1, wherein the DNA polymerase is a thermo-stable DNA polymerase.

8. The method for isothermal detection of target DNA according to claim 7, wherein the thermo-stable DNA polymerase is selected from the group consisting of Bst DNA polymerase, exo(-) vent DNA polymerase, exo(-) Deep vent DNA polymerase, exo(-) Pfu DNA polymerase, Bca DNA polymerase, and phi-29 DNA polymerase.

9. The method for isothermal detection of target DNA according to claim 1, wherein the RNase is RNase H.

10. The method for isothermal detection of target DNA according to claim 1, wherein the DNA-RNA-DNA hybrid primer consists of 32∼66 bases in length.

11. The method for isothermal detection of target DNA according to claim 11, wherein the DNA-RNA-DNA hybrid primer consists the both DNA regions of the DNA-RNA-DNA hybrid primer are 15∼30 bases in lengths, and the RNA region of the DNA-RNA-DNA hybrid primer is 1∼6 bases in length.

12. The method for isothermal detection of target DNA according to claim 1, wherein the DNA-RNA-DNA hybrid signal probe consists of 18∼38 bases in length.

13. The method for isothermal detection of target DNA according to claim 12, wherein the DNA-RNA-DNA hybrid signal probe consists the both DNA regions of the DNA-RNA-DNA hybrid primer are 8∼16 bases in lengths, and the RNA region of the DNA-RNA-DNA hybrid primer is 1∼6 bases in length.

14. The method for isothermal detection of target DNA according to claim 1, wherein the DNA-RNA-DNA hybrid signal probe is labeled with markers at the both ends thereof

15. The method for isothermal detection of target DNA according to claim 14, wherein the markers are selected from the group consisting of fluorescence, and quencher.

16. The method for isothermal detection of target DNA according to claim 1, wherein the isothermal detection is carried out at a temperature of 30 ∼ 75°C.
